(19) Europäisches Patentamt
European Patent Office
Office européen des brevets

(11) Veröffentlichungsnummer: **0 149 235**
**A1**

(12) # EUROPÄISCHE PATENTANMELDUNG

(21) Anmeldenummer: 84116289.4

(22) Anmeldetag: 24.12.84

(51) Int. Cl.⁴: C 07 D 249/08
C 07 D 233/60, A 01 N 43/653
A 01 N 43/50

(30) Priorität: 19.01.84 DE 3401714

(43) Veröffentlichungstag der Anmeldung:
24.07.85 Patentblatt 85/30

(84) Benannte Vertragsstaaten:
AT BE CH DE FR GB IT LI NL SE

(71) Anmelder: CELAMERCK GmbH & Co. KG
Binger Strasse 173
D-6507 Ingelheim am Rhein(DE)

(72) Erfinder: Curtze, Jürgen, Dr.
Rheingaublick 6
D-6222 Geisenheim-Johannisberg(DE)

(72) Erfinder: Stransky, Werner, Dr.
Im Hippel 24
D-6535 Gau-Algesheim(DE)

(72) Erfinder: Mengel, Rudolf, Dr.
Im Herzenacker 32
D-6535 Gau-Algesheim(DE)

(72) Erfinder: Becher, Heinz-Manfred, Dr.
Pfarrer-Heberer-Strasse 5
D-6530 Bingen(DE)

(72) Erfinder: Drandarevski, Christo, Dr.
Boehringerstrasse 8
D-6507 Ingelheim(DE)

(72) Erfinder: Lust, Sigmund, Dr.
Klappacher Strasse 2f
D-6100 Darmstadt(DE)

(54) **Neuartige Azole mit Enoletherstruktur, ihre Herstellung und Verwendung.**

(57) Die Erfindung betrifft neue Verbindungen der Formel I

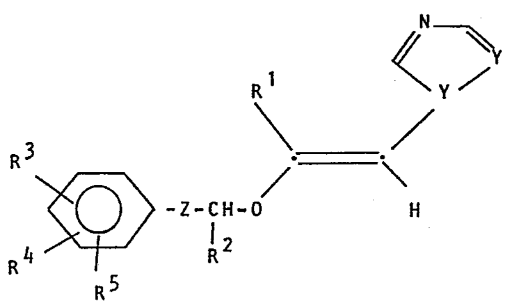

ihre Verwendung als Biozide und ihre Herstellung nach an sich bekannten Verfahren.

EP 0 149 235 A1

Die Erfindung betrifft eine neue Klasse von Imidazolen und Triazolen mit Enoletherstruktur, die der allgemeinen Formel

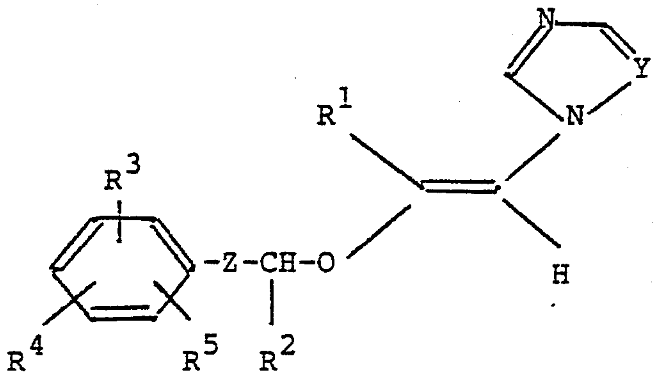

I

entsprechen, sowie deren Säureadditionssalze und Metallkomplexe. Die Erfindung betrifft ferner mikrobizide Mittel, die diese Verbindungen als Wirkstoff enthalten, die Herstellung der erfindungsgemäßen Verbindungen und mikrobizider Mittel, und die Verwendung der Wirkstoffe oder der Mittel zur Bekämpfung von schädlichen Mikroorganismen.

In der allgemeinen Formel I bedeuten

$R^1$ Niederalkyl, nicht jedoch $\alpha$-monoverzweigtes Niederalkyl

$R^2$ Wasserstoff oder Niederalkyl,

$R^3$ - $R^5$, die gleich oder verschieden sein können, Wasserstoff, Halogen, gegebenenfalls mit Halogen substituierte Niederalkyl und/oder Niederalkoxyreste, gegebenenfalls mit Halogen, Niederalkyl und/oder Niederalkoxy substituierte Phenyl-, Phenoxy- oder Phenylthioreste,

0149235

Y CH oder N und

Z Sauerstoff oder Schwefel,

Unter den Alkylketten in den Resten Niederalkyl bzw. Niederalkoxy sind geradkettige oder verzweigte $C_1$-$C_6$-Alkyle, vorzugsweise Methyl, Ethyl, n-Propyl, n-Butyl, und/oder tert.-Butyl zu verstehen. Unter Halogen sind Fluor, Chlor, Brom und/oder Jod, vorzugsweise Brom und/oder Chlor zu verstehen.

Die Verbindungen der Formel I sind stabile Öle, Harze oder Feststoffe mit wertvollen mikrobiziden und mikrostatischen Eigenschaften. Einzusetzen sind sie zur Verhütung von Mikroorganismenbefall und zur Behandlung von befallenen Pflanzen. Sie sind problemlos in der Anwendung und in weiten Anwendungskonzentrationen äußerst mikrobizid, beispielsweise fungistatisch oder fungizid wirksam.

Besonders bevorzugt sind Verbindungen der allgemeinen Formel I, in der

$R^1$ tert.-Butyl und n-Propyl

$R^2$ Wasserstoff,

$R^3$-$R^5$, die gleich oder verschieden sein können, Wasserstoff oder Halogen,

Y N und

Z Sauerstoff oder Schwefel bedeuten.

Zu den erfindungsgemäßen Substanzen gehören neben den freien Verbindungen auch deren Säureadditionssalze und Metallkomplexe.

Die für die Salzbildung geeigneten Säuren sind beispielsweise Halogenwasserstoff, Schwefel-, Phosphor- oder Salpetersäure als anorganische Salzbildner, und Essig-, Trifluoressig-, Oxal-, Benzolsulfon-, oder Salicylsäure als organische Salzbildner. Metallkomplexe der allgemeinen Formel I werden erhalten mit anorganischen oder organischen Metallsalzen; beispielsweise mit Halogeniden, Nitraten, Sulfaten oder Acetaten, Trihalogenacetaten, Tartraten, Benzoaten bevorzugt des Kupfers.

Die erfindungsgemäßen Verbindungen der Formel I können hergestellt werden, indem man eine Azolylverbindung der Formel

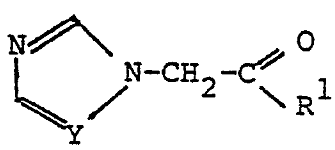

II

worin $R^1$ und Y die obigen Bedeutungen besitzen, mit Verbindungen der allgemeinen Formel III

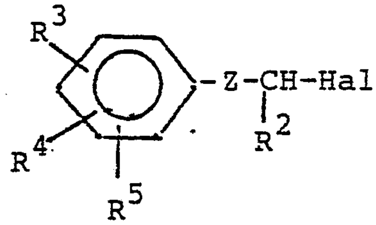

III

in der

Hal für Fluor, Chlor, Brom, Jod und $R^2$ - $R^5$ und Z für die obigen Bedeutungen stehen, in Gegenwart einer Base und vorteilhafterweise in Anwesenheit eines polaren, aprotischen Lösungsmittels oder eines wässrig-organischen Zweiphasenmediums in Gegenwart eines üblichen Phasentransferkatalysators reagieren läßt und die freien Verbindungen gemäß Formel I gegebenenfalls in die Salze oder Metallkomplexe überführt.

Als geeignete Basen haben sich anorganische Basen, wie Oxide, Hydroxide, Hydride, Carbonate und Hydrogencarbonate von Alkali-und Erdalkalimetallen, sowie organische Basen, wie tertiäre Amine herausgestellt.

Geeignete Lösungsmittel sind beispielsweise Dimethylsulfoxid, Dimethylformamid, Benzol, Toluol, sowohl alleine als auch in der Mischung, und mit Wasser nicht mischbare aliphatische und aromatische Kohlenwasserstoffe wie Pentan, Cyclohexan, Ligroin usw.

Die bei der Reaktion im Zweiphasensystem notwendigen Phasentransferkatalysatoren können beispielsweise Tetraalkylammoniumhalogenide, -hydrogensulfate, -hydroxide oder Phosphonium-Salze sein.

Die Reaktionstemperaturen liegen im allgemeinen zwischen $0^{\circ}$ und $120^{\circ}C$, vorzugsweise zwischen $20^{\circ}C$ und $80^{\circ}C$.

Die Ausgangsverbindungen sind bekannt oder werden nach bekannten Literaturvorschriften erhalten.

Bei der Synthese der Verbindungen der allgemeinen Formel I können zwei unterschiedliche geometrische Formen entstehen:

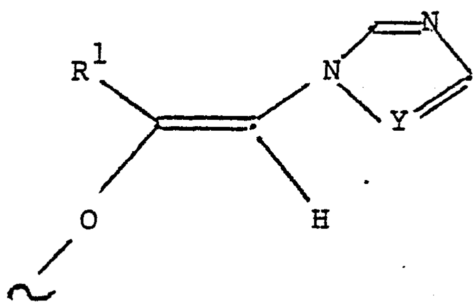

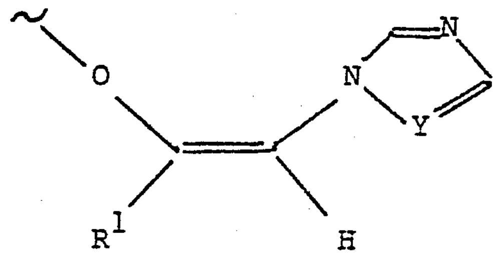

E-Isomer  Z-Isomer

Im allgemeinen entstehen Gemische beider Formen, die nach üblichen Methoden, z.B. Umkristallisation oder Chromatographie aufgetrennt werden können, wonach durch $^{1}H$-NMR-Daten mittels Verschiebungsreagenzien eine eindeutige Strukturzuordnung erfolgen kann.

Beide Isomere zeigen unterschiedliche mikrobizide Aktivität. Wenn nicht ausdrücklich erwähnt, beziehen sich die Daten und Angaben einer Verbindung gemäß Formel I stets auf die Gemische der beiden Isomeren.

Überraschenderweise zeigen die erfindungsgemäßen Verbindungen ein äußerst günstiges Mikrobizid-Spektrum gegen phytopathogene Pilze und Bakterien. Sie besitzen kurative, präventive und systemische Eigenschaften und lassen sich zum Schutz von Kulturpflanzen einsetzen.

Erfindungsgemäße Wirkstoffe lassen sich besonders vorteilhaft gegen folgende Klassen phytopathogener Pilze einsetzen: Ascomyceten, Basidiomyceten, Fungi imperfecti und Phycomyceten.

Folgende Pflanzenarten kommen als behandlungsfähig in Betracht: Getreide, Kern-, Stein- und Beerenobst, Hülsenfrüchte, Ölkulturen, Gurkengewächse, Fasergewächse, Citrusfrüchte, Gemüsearten, Lorbeergewächse, Mais, Tabak, Nüsse, Kaffee, Zuckerrohr, Tee, Weinreben, Hopfen, Bananen und Naturkautschukgewächse sowie Compositen.

Wirkstoffe der allgemeinen Formel I können gleichzeitig oder nacheinander mit weiteren Wirkstoffen auf die zu behandelnde Fläche oder an die Pflanze gegeben werden, wobei diese weiteren Wirkstoffe Präparate, die das Pflanzenwachstum beeinflussen oder Pflanzenschutzmittel sein können.

Für die Anwendung werden die erfindungsgemäßen Verbindungen, gewünschtenfalls auch die Salze, in üblicher Weise mit Hilfs- und/oder Trägerstoffen zu gebräuchlichen Formen von Schädlingsbekämpfungsmitteln verarbeitet, z.B. zu Lösungen, Lösungs- bzw. Emulsionskonzentraten, Suspensionspulvern, Stäuben, Emulsionen. Die Konzentrate werden vor der Anwendung gegebenenfalls mit Wasser verdünnt, so daß Spritzbrühen mit einem Wirkstoffgehalt zwischen etwa 0,005 und 1 Gewichtsprozent erhalten werden. Bei der Anwendung als Low-Volume- oder Ultra-Low-Volume-Formulierung kann der Wirkstoffgehalt auch erheblich höher sein (bis 20 bzw. 50 Gewichtsprozent).

Bevorzugte Applikationsformen sind die Blattapplikation, Bodenapplikation und das Coating der Samenkörner.

Beispiele für erfindungsgemäße Formulierungen:

1. Suspensionspulver

20 Gew.-Teile einer Verbindung nach Formel I
20 Gew.-Teile Kaolin
5 Gew.-Teile Natriumsulfat
2 Gew.-Teile Schlämmkreide
9 Gew.-Teile Calciumligninsulfonat (Dispergiermittel)
1 Gew.-Teil Diisobutylnaphthalin-natriumsulfonat (Netzmittel)
43 Gew.-Teile Kieselkreide

Die Bestandteile werden vermahlen und das Mittel wird für die Anwendung in so viel Wasser suspendiert, daß die Wirkstoffkonzentration etwa 0,005 bis 0,5 Gewichtsprozent beträgt.

2. Emulsionskonzentrat

15 Gew.-Teile einer Verbindung nach Formel I
10 Gew.-Teile Dodecylbenzolsulfonsäure-triäthylaminsalz
75 Gew.-Teile Dimethylformamid

## Beispiel 1

**1-[1-(1,2,4-Triazolyl)]-2-[(4-chlorphenoxy)-methoxy]-3,3-dimethyl-buten-1**

2,7 g Natriumhydrid (0,117 Mol) werden in 22 ml Dimethylformamid suspendiert,nun wird unter Rühren innerhalb von 15 Minuten eine Lösung von 16,7 g (0,1 Mol) 3,3-Dimethyl-1-(1,2,4-triazol-1-yl)-2-butanon in 22 ml Dimethylformamid zugetropft. Die Lösung wird 20 Stunden bei Raumtemperatur gerührt, anschließend werden 17,7 g (0,1 Mol) Chlormethyl-4-chlorphenylether in 22 ml Dimethylformamid unter starker Kühlung innerhalb von 20 Minuten hinzugefügt. Die Lösung wird 10 Stunden bei 10°C gerührt.

Anschließend wird in 20 ml Wasser gegossen. Es wird mit Methylenchlorid extrahiert. Das Methylenchlorid wird getrocknet und eingedampft. Der Rückstand wird über eine Kieselgelsäule mit Toluol/Aceton 7:3 gereinigt. Es werden 15,3 g eines allmählich erstarrenden Öls erhalten.
Smp.: 59-60°C.

$^1H$-NMR; ($CDCl_3$): $\delta$ = 1,18 s; 9H (tert.Butyl); 5,11 s, 2H($O-CH_2-O$-Aryl); 6,51 s, 1H (>C=CH-Triazol); 6,91 - 7,31 m, 4H (Aryl); 8,06 s, 1H (Triazol) 8,34 s, 1H (Triazol)

Analyse: $C_{15}H_{18}N_3O_2Cl$ (307,5)

| | C | H | N |
|---|---|---|---|
| Ber.: | 58,54 | 5,85 | 13,66 |
| gef.: | 58,31 | 5,78 | 13,32 |

Beispiel 2

1-/1-(1,2,4-Triazolyl)7-2-/(4-chlorphenoxy)-methylthio7-3,3-dimethyl-buten-1

Das im Beispiel 1 beschriebene Verfahren wird mit der äquivalenten Menge Chlormethyl-4-chlorphenylthioether anstelle des Chlormethyl-4-chlorphenylether durchgeführt. Die Reinigung erfolgt an Kieselgel mit Toluol/Essigester 1 : 1 ($R_F$ = 0,33).

$^1$H-NMR; ($CDCl_3$): $\delta$ = 1,15 s, 9H (tert.Butyl); 4,78 s, 2H(O-$CH_2$-S-Aryl); 6,42 s, 1H (>C=CH-Triazol); 7,34 m, 4H(Aryl); 8,0 s, 1H (Triazol); 8,0 s, 1H (Triazol); 8,3 s, 1H (Triazol)

Beispiel 3

1-/1-(1,2,4-Triazolyl)7-2-/(4-chlorphenoxy)-methoxy7-penten—1

Analog zu Beispiel 1 erhält man aus 15,3 g (0,1 Mol) 1-/-(1,2,4-Triazolyl)7-pentanon-2 und 17,7 g (0,1 Mol) Chlormethyl-4-chlorphenylether nach extraktiver Aufarbeitung und Vakuumdestillation 23,8 g (81 % d.Th.) der gewünschten Verbindung.
$Kp_{0.5}$: 178 - 179°C.

In analoger Weise werden die in der Tabelle formelmäßig aufgeführten Verbindungen erhalten:

| Nr. | $R^1$ | $R^2$ | $R^3$ | $R^4$ | $R^5$ | Y | Z | |
|---|---|---|---|---|---|---|---|---|
| 1 | tert.Butyl | H | H | H | H | N | O | |
| 2 | " " | H | 2-Cl | H | H | N | O | $R_f$: 0,28* |
| 3 | " " | H | H | H | 4-F | N | O | |
| 4 | " " | H | H | H | 4-J | N | O | |
| 5 | " " | H | H | H | 4-Br | N | O | |
| 6 | " " | H | H | H | 4-Cl | N | O | |
| 7 | " " | H | H | 3-$CF_3$ | H | N | O | |
| 8 | " " | H | 2-Cl | H | 4-Cl | N | O | Fp: 95-96°C |
| 9 | " " | H | 2-Cl | H | 5-Cl | N | O | |
| 10 | " " | H | 2-Cl | H | 6-Cl | N | O | $R_f$: 0,43** |
| 11 | " " | H | H | 3-Cl | 4-Cl | N | O | Fp. 80-82°C |
| 12 | " " | H | 2-Cl | 5-Cl | 4-Br | N | O | |
| 13 | " " | H | 2-Cl | 4-Cl | 6-Cl | N | O | $R_f$: 0,40* |
| 14 | " " | $CH_3$ | H | H | 4-Cl | N | O | |
| 15 | " " | H | H | H | 4-Cl | CH | O | |
| 16 | " " | H | H | 3-Cl | 4-Cl | CH | O | |
| 17 | " " | H | 2-Cl | 4-Cl | H | CH | O | Fp: 81°C |
| 18 | " " | H | 4-$C_6H_5$ | H | H | N | O | |
| 19 | " " | H | 4-$C_6H_5$ | H | H | CH | O | |
| 20 | " " | H | 4-Br | H | H | N | O | |
| 21 | " " | H | 4-CN | H | H | N | O | |
| 22 | " " | H | 4-$NO_2$ | H | H | N | O | |
| 23 | " " | H | 3-$CH_3$ | 4-$CH_3$ | H | N | O | |
| 24 | " " | H | 3-$CH_3$ | 4-$CH_3$ | H | CH | O | |
| 25 | " " | H | 4-F | H | H | N | O | |
| 26 | " " | H | 3-$CH_3$ | 4-Cl | H | N | O | Fp: 80-87°C |
| 27 | " " | H | 3-$CH_3$ | 4-Cl | H | CH | O | |
| 28 | " " | H | 3-$CH_3$ | 4-$SCH_3$ | H | N | O | |

| Nr. | $R^1$ | $R^2$ | $R^3$ | $R^4$ | $R^5$ | Y | Z | |
|---|---|---|---|---|---|---|---|---|
| 29 | tert.Butyl | H | 4-OMe | H | H | N | O | |
| 30 | " " | H | 3-Cl | 5-Cl | H | N | O | |
| 31 | " " | H | 3-$OCH_3$ | 4-$OCH_3$ | H | N | O | |
| 32 | " " | H | 3-Cl | 4-Cl | 5-Cl | N | O | |
| 33 | n-Propyl | H | 2-Cl | H | 4-Cl | N | O | Fp: 74-75°C |
| 34 | " " | H | 2-Cl | 4-Cl | 6-Cl | N | O | $R_f$: 0,47* |
| 35 | " " | H | 2-Cl | H | 4-Cl | CH | O | |
| 36 | " " | H | 2-Cl | 4-Cl | 6-Cl | CH | O | $R_f$: 0,36*** |
| 37 | " " | H | H | H | 4-Cl | N | S | |
| 38 | " " | H | 2-Cl | 6-Cl | H | N | O | Fp: 63-64°C |
| 39 | " " | H | 4-$C_6H_5$ | H | H | N | O | |
| 40 | n-Butyl | H | 3-Cl | 4-Cl | H | N | O | |
| 41 | $C_2H_5$ | H | 3-Cl | 4-Cl | H | N | O | |
| 42 | $C_2H_5$ | H | 3-Me | 4-Me | H | N | O | |
| 43 | $CH_3$ | H | 3-Cl | 4-Cl | H | N | O | |

* Laufmittel: $CH_3CO_2$ Et/ Toluol (1:1)

** Laufmittel: i-$C_3H_7OH$ / Benzin 80-110 (1:4)

*** Laufmittel: i-$C_3H_7OH$/Benzin 80-110 (1:3)

## Patentansprüche

1) Verbindungen der allgemeinen Formel I

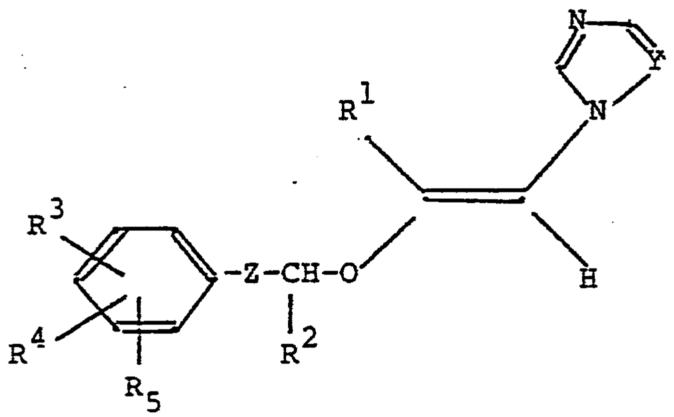

I

in der

$R^1$ Niederalkyl, außer $C_1$-$C_6$-α-monoverzweigtes Alkyl

$R^2$ Wasserstoff oder Niederalkyl,

$R^3$ - $R^5$, die gleich oder verschieden sein können, Wasserstoff, Halogen, gegebenenfalls mit Halogen substituierte Niederalkyl- und/oder Niederalkoxyreste, gegebenenfalls mit Halogen, Niederalkyl und/oder Niederalkoxy substituierte Phenyl-, Phenoxy-oder Phenolthioreste,

Y CH oder N und

Z Sauerstoff oder Schwefel bedeuten unter Einbeziehung ihrer Säureadditionssalze und Metallkomplexe.

Unter den Alkylketten in den Resten Niederalkyl bzw. Niederalkoxy sind geradkettige oder verzweigte $C_1$-$C_6$-Alkyle, vorzugsweise Methyl, Ethyl, n-Propyl, n-Butyl, und/oder tert.-Butyl zu verstehen.
Unter Halogen sind Fluor, Chlor, Brom und/oder Jod, vorzugsweise Brom und/oder Chlor zu verstehen.

2) Verbindungen gemäß Anspruch 1, dadurch gekennzeichnet, daß Y Stickstoff und Z Sauerstoff bedeuten.

3) Verbindungen gemäß Anspruch 1, dadurch gekennzeichnet, daß Y Stickstoff und Z Schwefel bedeuten.

4) Verbindungen gemäß einem der vorhergehenden Ansprüche, dadurch gekennzeichnet, daß $R^1$ tert.-Butyl oder n-Propyl und $R^3$ - $R^5$, die gleich oder verschieden sein können, Wasserstoff und/oder Halogen bedeuten.

5) Verfahren zur Herstellung von Verbindungen der allgemeinen Formel I, dadurch gekennzeichnet, daß man eine Verbindung der Formel II

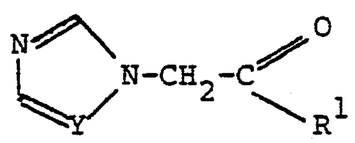

II

worin $R^1$ und Y die obigen Bedeutungen haben, mit einer Verbindung der Formel III

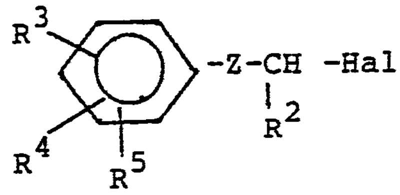

III

worin Z und $R^2$ - $R^5$ die obigen Bedeutungen besitzen und Hal für Fluor, Chlor, Brom oder Jod steht, in Gegenwart einer Base umsetzt und die erhaltenen Produkte in üblicher Weise isoliert und reinigt.

6) Biozide Mittel, dadurch gekennzeichnet, daß sie neben üblichen Hilfs- und/oder Trägerstoffen einen Gehalt an mindestens einer Verbindung nach Anspruch 1 besitzen.

7) Biozides Mittel gemäß Anspruch 6, dadurch gekennzeichnet, daß es mindestens eine Verbindung gemäß Anspruch 4 enthält.

8) Verwendung von Verbindungen gemäß Anspruch 1 - 4 zur Bekämpfung und/oder zur Verhütung eines Befalls von Mikroorganismen.

9) Verwendung gemäß Anspruch 7, dadurch gekennzeichnet, daß die Mikroorganismen phytopathogene Pilze sind.

10) Verwendung der Mittel gemäß Anspruch 6 und 7 zur Bekämpfung und/oder zur Verhütung eines Befalls von Mikroorganismen.

Europäisches Patentamt

**0149235**

Nummer der Anmeldung

# EUROPÄISCHER RECHERCHENBERICHT

EP 84116289.4

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
| A | DE - A1 - 3 126 478 (BASF)<br>* Formel 1 *<br>-- | 1 | C 07 D 249/08<br>C 07 D 233/60<br>A 01 N 43/653<br>A 01 N 43/50 |
| A | EP - A3 - 0 060 223 (CIBA)<br>* Zusammenfassung *<br>-- | 1,7 | |
| A | EP - A1 - 0 079 856 (CIBA)<br>* Formel I *<br>-- | 1,7 | |
| A | EP - A3 - 0 072 580 (BAYER)<br>* Zusammenfassung *<br>-- | 1 | |
| A | CHEMICAL ABSTRACTS, Vol. 95, No. 19, 9. November 1981, Columbus, Ohio, USA<br>LEBEDEVA, N.P.; STOTSKII, A.A.; FOMINA, V.V. Nitration of N-alkenyl-1,2,4-triazoles. Seite 734, Spalte 1, Zusammenfassung-Nr. 169 096h<br>& Zh. Org. Khim. 1981, 17(8), 1786-7<br>---- | 1 | RECHERCHIERTE SACHGEBIETE (Int. Cl.4)<br>C 07 D 249/00<br>C 07 D 233/00 |

Der vorliegende Recherchenbericht wurde für alle Patentansprüche erstellt.

| Recherchenort | Abschlußdatum der Recherche | Prüfer |
|---|---|---|
| WIEN | 18-04-1985 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82